# EUROPEAN PATENT APPLICATION

(11) **EP 4 592 321 A1**
(43) Date of publication of application: **30.07.2025**
(21) Application number: 23867511.0
(22) Date of filing: 19.09.2023
(51) Int. Cl.: C07K 16/32, C07K 16/28, A61K 39/395, C12N 15/13, A61P 35/00

(54) **ANTI-HER2 ANTIBODIES AND USE THEREOF**

(30) Priority: 20.09.2022 CN 202211142864
(71) Applicant: Biotheus Inc., Tangjiawan Town, Xiangzhou District Zhuhai, Guangdong 519080 (CN)
(72) Inventor: WU, Fan, Zhuhai, Guangdong 519080 (CN); CHEN, Liandi, Zhuhai, Guangdong 519080 (CN); MIAO, Xiaoniu, Zhuhai, Guangdong 519080 (CN); XU, Yingda, Zhuhai, Guangdong 519080 (CN)
(74) Representative: Ipsilon
(86) International application number: PCT/CN2023/119763
(87) International publication number: WO 2024/061224

(57) **Abstract**

Provided are anti-HER2 antibodies and use thereof, and specifically provided are a series of anti-HER2 antibodies. The antibodies have high binding affinity for HER2, have cross-reaction activity with human and cynomolgus monkey HER2, and exhibit good thermal stability, and show excellent anti-tumor effect in various tumors (such as neurocytoma, breast cancer, ovarian cancer, gastric cancer, lung cancer, kidney cancer, intestinal cancer, pancreatic cancer, bladder cancer, and the like).

## Description

### Technical Field

The present invention relates to the field of antibody drugs, and specifically to anti-HER2 antibodies and uses thereof.

### Background Art

HER2, also known as ErbB2, belongs to the HER subfamily in the type I receptor tyrosine kinase family, and the subfamily also includes 3 other members: HER1 (ErbB 1 or EGFR), HER3 (ErbB3) and HER4 (ErbB4). The HER subfamily members can form homodimers and heterodimers, and HER2 is the strongest dimerization partner of other ErbB receptors. HER2 activation leads to receptor phosphorylation, which can trigger downstream signal cascade amplification through multiple signal pathways such as MAPK, PI3K/AKT, JAK/STAT and PKC, thereby playing an important regulatory role in cell proliferation, differentiation, development, adhesion and migration. Researchers have found that high expression of HER2 is associated with many tumors, such as neurocytoma, breast cancer, ovarian cancer, gastric cancer, lung cancer, kidney cancer, intestinal cancer, pancreatic cancer, bladder cancer, etc.

Currently, Trastuzumab and Pertuzumab are the main HER2 targeted therapeutic antibodies on the market. Trastuzumab recognizes HER2 extracellular domain IV, Pertuzumab recognizes HER2 extracellular domain II heterodimerization site, and they significantly improve the survival of patients. However, researchers also found that Trastuzumab has no therapeutic effect on many tumor patients with high HER2 expression.

Therefore, there is still a need in this field to develop new anti-HER2 antibodies.

### Contents of the present invention

The inventors of the present application have screened and obtained a series of anti-HER2 antibodies after extensive research, which have high binding affinity with HER2, cross-reactivity with human and cynomolgus monkey HER2, good thermal stability, and excellent anti-tumor effects in various tumors (e.g., neurocytoma, breast cancer, ovarian cancer, gastric cancer, lung cancer, kidney cancer, intestinal cancer, pancreatic cancer, bladder cancer, etc.). Therefore, the present invention provides the following aspects.

### Antibody or antigen-binding fragment thereof

In the first aspect, the present invention provides an antibody or antigen-binding fragment thereof capable of specifically binding to HER2, wherein the antibody or antigen-binding fragment thereof comprises:
(1) the following 3 heavy chain variable region (VH) complementarity determining regions (CDRs):
   (a) VH CDR1, which has a structure represented by GFNIKDTY (SEQ ID NO: 10);
   (b) VH CDR2, which has a structure represented by IYPTQGYT (SEQ ID NO: 11);
   (c) VH CDR3, which has a structure represented by SRWGGEGFYAMDY (SEQ ID NO: 12);
      and/or,
(2) the following 3 light chain variable region (VL) complementarity determining regions (CDRs):
   (d) VL CDR1, which has a structure represented by X₁X₂VQX₃A (SEQ ID NO: 33);
   (e) VL CDR2, which has a structure represented by SAS (SEQ ID NO: 19, SEQ ID NO: 23 or SEQ ID NO: 27);
   (f) VL CDR3, which has a structure represented by QQHX₄X₅TPPT (SEQ ID NO: 34);
      wherein:
      X₁ is selected from amino acid residues Q and N;
      X₂ is selected from amino acid residues N, Y and S;
      X₃ is selected from amino acid residues G and T;
      X₄ is selected from amino acid residues Y, F and S;
      X₅ is selected from amino acid residues S, M and T.

In some embodiments, the antibody or antigen-binding fragment thereof comprises:
a VH CDR1 as set forth in SEQ ID NO: 10, a VH CDR2 as set forth in SEQ ID NO: 11, and a VH CDR3 as set forth in SEQ ID NO: 12; and,
a VL CDR1 as set forth in any one of SEQ ID NOs: 18, 22 and 26, a VL CDR2 as set forth in any one of SEQ ID NOs: 19, 23 and 27, and a VL CDR3 as set forth in any one of SEQ ID NOs: 20, 24 and 28.

In some embodiments, the antibody or antigen-binding fragment thereof comprises: a VH CDR1 as set forth in SEQ ID NO: 10, a VH CDR2 as set forth in SEQ ID NO: 11, and a VH CDR3 as set forth in SEQ ID NO: 12; and, a VL CDR1 as set forth in SEQ ID NO: 18, a VL CDR2 as set forth in SEQ ID NO: 19, and a VL CDR3 as set forth in SEQ ID NO: 20.

In some embodiments, the antibody or antigen-binding fragment thereof comprises: a VH CDR1 as set forth in SEQ ID NO: 10, a VH CDR2 as set forth in SEQ ID NO: 11, and a VH CDR3 as set forth in SEQ ID NO: 12; and, a VL CDR1 as set forth in SEQ ID NO: 22, a VL CDR2 as set forth in SEQ ID NO: 23, and a VL CDR3 as set forth in SEQ ID NO: 24.

In some embodiments, the antibody or antigen-binding fragment thereof comprises: a VH CDR1 as set forth in SEQ ID NO: 10, a VH CDR2 as set forth in SEQ ID NO: 11, and a VH CDR3 as set forth in SEQ ID NO: 12; and, a VL CDR1 as set forth in SEQ ID NO: 26, a VL CDR2 as set forth in SEQ ID NO: 27, and a VL CDR3 as set forth in SEQ ID NO: 28.

In some embodiments, the antibody or antigen-binding fragment thereof comprises: a VH having a sequence as set forth in SEQ ID NO: 9 or a variant thereof, and a VL having a sequence as set forth in any one of SEQ ID NOs: 17, 21 and 25 or a variant thereof;
wherein, the variant has a sequence having a substitution, deletion or addition of one or more amino acids (e.g., a substitution, deletion or addition of 1, 2, 3, 4 or 5 amino acids), or a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived; preferably, the substitution is a conservative substitution.

In some embodiments, the antibody or antigen-binding fragment thereof comprises: a VH having a sequence as set forth in SEQ ID NO: 9, and a VL having a sequence as set forth in SEQ ID NO: 17.

In some embodiments, the antibody or antigen-binding fragment thereof comprises: a VH having a sequence as set forth in SEQ ID NO: 9, and a VL having a sequence as set forth in SEQ ID NO: 21.

In some embodiments, the antibody or antigen-binding fragment thereof comprises: a VH having a sequence as set forth in SEQ ID NO: 9, and a VL having a sequence as set forth in SEQ ID NO: 25.

In some embodiments, the antibody or antigen-binding fragment thereof further comprises a constant region derived from a human immunoglobulin.

In some embodiments, the antibody or antigen-binding fragment thereof has a heavy chain constant region derived from a human immunoglobulin (e.g., IgG1, IgG2, IgG3, or IgG4).

In some embodiments, the antibody or antigen-binding fragment thereof has a light chain constant region derived from a human immunoglobulin (e.g., x or λ).

In some embodiments, the antibody or antigen-binding fragment thereof further comprises a heavy chain constant region as set forth in SEQ ID NO: 29 and/or a light chain constant region as set forth in SEQ ID NO: 31.

In some embodiments, the antigen-binding fragment is selected from the group consisting of Fab, Fab', (Fab')₂, Fv, disulfide-linked Fv, scFv, diabody and single-domain antibody (sdAb).

The antibodies of the present invention can be prepared by various methods known in the art, such as by genetic engineering recombinant technology. For example, DNA molecules encoding the heavy chain and light chain of the antibodies of the present invention are obtained by chemical synthesis or PCR amplification. The obtained DNA molecules are inserted into expression vectors and then transfected into host cells. Then, the transfected host cells are cultured under specific conditions and the antibodies of the present invention are expressed.

The antigen-binding fragments of the present invention can be obtained by hydrolyzing intact antibody molecules (see, Morimoto et al., J. Biochem. Biophys. Methods 24: 107-117 (1992) and Brennan et al., Science 229: 81 (1985)). In addition, these antigen-binding fragments can also be directly produced by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab or F(ab')₂ fragments can also be directly isolated from recombinant host cell culture fluid. Ordinary technicians in this field are fully aware of other techniques for preparing these antigen-binding fragments.

The CDRs of the present invention are delimited according to the IMGT delimiting website http: //aligncdr.labshare.cn/aligncdr/abrsa.php.

### single-arm antibody

In the second aspect, the present invention provides a single-arm antibody, which comprises:
(1) a first peptide chain, wherein the first peptide chain comprises the following 3 heavy chain variable region (VH) complementarity determining regions (CDRs):
   (a) VH CDR1, which has a structure represented by GFNIKDTY (SEQ ID NO: 10);
   (b) VH CDR2, which has a structure represented by IYPTQGYT (SEQ ID NO: 11);
   (c) VH CDR3, which has a structure represented by SRWGGEGFYAMDY (SEQ ID NO: 12);
(2) a second peptide chain, wherein the second peptide chain comprises the following 3 light chain variable region (VL) complementarity determining regions (CDRs):
   (d) VL CDR1, which has a structure represented by X₁X₂VQX₃A (SEQ ID NO: 33);
   (e) VL CDR2, which has a structure represented by SAS (SEQ ID NO: 19, SEQ ID NO: 23 or SEQ ID NO: 27);
   (f) VL CDR3, which has a structure represented by QQHX₄X₅TPPT (SEQ ID NO: 34);
      and,
(3) a third peptide chain, wherein the third peptide chain is capable of forming a dimer with the first peptide chain;
   wherein:
   X₁ is selected from amino acid residues Q and N;
   X₂ is selected from amino acid residues N, Y and S;
   X₃ is selected from amino acid residues G and T;
   X₄ is selected from amino acid residues Y, F and S;
   X₅ is selected from amino acid residues S, M and T.

In some embodiments, the first peptide chain comprises a VH CDR1 as set forth in SEQ ID NO: 10, a VH CDR2 as set forth in SEQ ID NO: 11, and a VH CDR3 as set forth in SEQ ID NO: 12.

In some embodiments, the second peptide chain comprises a VL CDR1 as set forth in any one of SEQ ID NOs: 18, 22 and 26, a VL CDR2 as set forth in any one of SEQ ID NOs: 19, 23 and 27, and a VL CDR3 as set forth in any one of SEQ ID NOs: 20, 24 and 28.

In some embodiments, the second peptide chain comprises a VL CDR1 as set forth in SEQ ID NO: 18, a VL CDR2 as set forth in SEQ ID NO: 19, and a VL CDR3 as set forth in SEQ ID NO: 20.

In some embodiments, the second peptide chain comprises a VL CDR1 as set forth in SEQ ID NO: 22, a VL CDR2 as set forth in SEQ ID NO: 23, and a VL CDR3 as set forth in SEQ ID NO: 24.

In some embodiments, the second peptide chain comprises a VL CDR1 as set forth in SEQ ID NO: 26, a VL CDR2 as set forth in SEQ ID NO: 27, and a VL CDR3 as set forth in SEQ ID NO: 28.

In some embodiments, the first peptide chain comprises a heavy chain variable region (VH) having a sequence as set forth in SEQ ID NO: 9 or a variant thereof, wherein the variant has a sequence having a substitution, deletion or addition of one or more amino acids (e.g., a substitution, deletion or addition of 1, 2, 3, 4 or 5 amino acids), or a sequence identity of at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived; preferably, the substitution is a conservative substitution.

In some embodiments, the first peptide chain comprises a heavy chain variable region (VH) having a sequence as set forth in SEQ ID NO: 9.

In some embodiments, the second peptide chain comprises a light chain variable region (VL) having a sequence as set forth in any one of SEQ ID NOs: 17, 21 and 25 or a variant thereof, wherein the variant has a sequence having a substitution, deletion or addition of one or more amino acids (e.g., a substitution, deletion or addition of 1, 2, 3, 4 or 5 amino acids), or a sequence identity of at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived; preferably, the substitution is a conservative substitution.

In some embodiments, the second peptide chain comprises a light chain variable region (VL) having a sequence as set forth in any one of SEQ ID NOs: 17, 21 and 25.

In some embodiments, the second peptide chain further comprises a constant region derived from a human immunoglobulin.

In some embodiments, the second peptide chain comprises a light chain constant region derived from a human immunoglobulin (e.g., κ or λ).

In some embodiments, the second peptide chain comprises a light chain constant region as set forth in SEQ ID NO: 31.

In some embodiments, the first peptide chain further comprises a constant region derived from a human immunoglobulin. In some embodiments, the constant region derived from a human immunoglobulin is a heavy chain constant region derived from a human immunoglobulin (e.g., IgG1, IgG2, IgG3 or IgG4). In some embodiments, the heavy chain constant region has a first modification to promote dimerization of the first peptide chain and the third peptide chain.

In some embodiments, the third peptide chain comprises an Fc domain monomer. In some embodiments, the Fc domain monomer is an Fc domain monomer of IgG, such as an Fc domain monomer of IgG1, IgG2, IgG3 or IgG4. In some embodiments, the Fc domain monomer has a second modification to promote dimerization of the third peptide chain and the first peptide chain.

In some embodiments, any one of the first modification and the second modification is a "knob" modification, and the other is a "hole" modification to form a "knob-into-hole" modification to promote dimerization of the first peptide chain and the third peptide chain.

In some embodiments, the first modification is a "knob" modification, and the second modification is a "hole" modification to form a "knob-into-hole" modification to promote dimerization of the first peptide chain and the third peptide chain.

In some embodiments, the heavy chain constant region comprises an amino acid sequence as set forth in SEQ ID NO: 30, and the Fc domain monomer comprises an amino acid sequence as set forth in SEQ ID NO: 32.

The knob-into-hole technique is described in, for example, US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and introducing a corresponding cavity ("hole") in the interface of a second polypeptide, so that the protuberance can be placed in the cavity to promote heterodimer formation and hinder homodimer formation. The protuberance is constructed by replacing a small amino acid side chain from the interface of the first polypeptide with a larger side chain (e.g., tyrosine or tryptophan). A complementary cavity of the same or similar size as the protuberance is created in the interface of the second polypeptide by replacing a large amino acid side chain with a smaller amino acid side chain (e.g., alanine or threonine).

### Isolated nucleic acid molecule

In the third aspect, the present invention provides an isolated nucleic acid molecule, which encodes the antibody or antigen-binding fragment thereof of the first aspect, or heavy chain variable region and/or light chain variable region thereof; or, which encodes the single-arm antibody of the second aspect, or heavy chain variable region and/or light chain variable region thereof.

In some embodiments, the isolated nucleic acid molecule comprises a first nucleotide sequence encoding the heavy chain or heavy chain variable region of the antibody or antigen-binding fragment thereof of the present invention and a second nucleotide sequence encoding the light chain or light chain variable region of the antibody or antigen-binding fragment thereof, wherein the first nucleotide sequence and the second nucleotide sequence are present in the same or different isolated nucleic acid molecules. When the first nucleotide sequence and the second nucleotide sequence are present in different isolated nucleic acid molecules, the isolated nucleic acid molecule of the present invention comprises a first nucleic acid molecule comprising the first nucleotide sequence and a second nucleic acid molecule comprising the second nucleotide sequence.

In some embodiments, the isolated nucleic acid molecule comprises a first nucleotide sequence encoding the first peptide chain or heavy chain variable region thereof of the single-arm antibody of the present invention, a second nucleotide sequence encoding the second peptide chain or light chain variable region of the single-arm antibody, and a third nucleotide sequence encoding the third peptide chain of the single-arm antibody, wherein the first nucleotide sequence, the second nucleotide sequence, and the third nucleotide sequence are present in the same or different isolated nucleic acid molecules. When the first nucleotide sequence, the second nucleotide sequence, and the third nucleotide sequence are present in different isolated nucleic acid molecules, the isolated nucleic acid molecule of the present invention comprises a first nucleic acid molecule comprising the first nucleotide sequence, a second nucleic acid molecule comprising the second nucleotide sequence, and a third nucleic acid molecule comprising the third nucleotide sequence.

### Vector

In the fourth aspect, the present invention provides a vector, which comprises the nucleic acid molecule of the third aspect. In some embodiments, the vector is a cloning vector or an expression vector.

In some embodiments, the vector comprises a first nucleotide sequence encoding the heavy chain or heavy chain variable region of the antibody or antigen-binding fragment thereof of the present invention and a second nucleotide sequence encoding the light chain or light chain variable region of the antibody or antigen-binding fragment thereof, wherein the first nucleotide sequence and the second nucleotide sequence are present in the same or different vectors. When the first nucleotide sequence and the second nucleotide sequence are present in different vectors, the vector of the present invention comprises a first vector comprising the first nucleotide sequence and a second vector comprising the second nucleotide sequence.

In some embodiments, the vector comprises a first nucleotide sequence encoding the first peptide chain or heavy chain variable region thereof of the single-arm antibody of the present invention, a second nucleotide sequence encoding the second peptide chain or light chain variable region thereof of the single-arm antibody, and a third nucleotide sequence encoding the third peptide chain of the single-arm antibody, wherein the first nucleotide sequence, the second nucleotide sequence, and the third nucleotide sequence are present in the same or different vectors. When the first nucleotide sequence, the second nucleotide sequence, and the third nucleotide sequence are present in different vectors, the vector of the present invention comprises a first vector comprising the first nucleotide sequence, a second vector comprising the second nucleotide sequence, and a third vector comprising the third nucleotide sequence.

### Host cell

In the fifth aspect, the present invention provides a host cell, which comprises the nucleic acid molecule as described in the third aspect or the vector as described in the fourth aspect. Such host cells include, but are not limited to, prokaryotic cells such as bacterial cells (e.g., *Escherichia coli* cells), and eukaryotic cells such as fungal cells (e.g., yeast cells), insect cells, plant cells, and animal cells (e.g., mammalian cells, such as mouse cells, human cells, etc.), etc.

### Preparation method

In the sixth aspect, the present invention provides a method for preparing the antibody or antigen-binding fragment thereof of the first aspect or the single-arm antibody of the second aspect, which comprises culturing the host cell of the fifth aspect under conditions that allow the expression of the antibody or antigen-binding fragment thereof or the single-arm antibody, and recovering the antibody or antigen-binding fragment thereof or the single-arm antibody from a culture of the cultured host cell.

### Therapeutic use

In the seventh aspect, the present invention provides a pharmaceutical composition, which comprises the antibody or antigen-binding fragment thereof of the first aspect or the single-arm antibody of the second aspect, and optionally comprising pharmaceutically acceptable carriers and/or excipients.

In certain exemplary embodiments, the pharmaceutically acceptable carriers and/or excipients comprise a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

In the eighth aspect, the present invention provides the use of the antibody or antigen-binding fragment thereof of the first aspect, the single-arm antibody of the second aspect, the isolated nucleic acid molecule of the third aspect, the vector of the fourth aspect, or the host cell of the fifth aspect, in the manufacture of a medicament for activating HER2, increasing immune cell activity, enhancing immune response, and/or preventing and/or treating a tumor or infection in a subject.

In some embodiments, the immune cell is a T cell, a B cell, a DC cell, a macrophage, and/or, an NK cell.

In some embodiments, the immune response is a HER2-mediated immune response.

In some embodiments, the tumor is selected from a solid tumor or a blood tumor (e.g., leukemia, lymphoma, myeloma). In certain embodiments, the tumor is selected from a solid tumor or a lymphoma.

In some embodiments, the tumor is selected from the group consisting of neurocytoma, breast cancer, ovarian cancer, gastric cancer, lung cancer, kidney cancer, intestinal cancer, pancreatic cancer, bladder cancer, colorectal cancer, colon cancer, uterine/cervical cancer, prostate cancer, testicular cancer, esophageal cancer, gastrointestinal cancer, head and neck cancer, germ cell cancer, bone cancer, liver cancer, thyroid cancer, skin cancer, tumor of central nervous system, lymphoma, leukemia, myeloma, sarcoma, and melanoma.

In some embodiments, the infection is selected from the group consisting of viral infection, bacterial infection, fungal infection, and parasitic infection.

In some embodiments, the subject is a mammal, such as a human.

In some embodiments, the antibody or antigen-binding fragment thereof or the single-arm antibody is used alone or in combination with an additional pharmaceutically active agent.

In the ninth aspect, the present invention provides a method for enhancing immune response, and/or preventing and/or treating a tumor or infection in a subject, the method comprises: administering to the subject in need thereof an effective amount of the antibody or antigen-binding fragment thereof of the first aspect or the single-arm antibody of the second aspect or the pharmaceutical composition of the seventh aspect.

In some embodiments, the immune response is a HER2-mediated immune response.

In some embodiments, the tumor is selected from a solid tumor or a blood tumor (e.g., leukemia, lymphoma, myeloma). In certain embodiments, the tumor is selected from a solid tumor or a lymphoma.

In some embodiments, the tumor is selected from the group consisting of neurocytoma, breast cancer, ovarian cancer, gastric cancer, lung cancer, kidney cancer, intestinal cancer, pancreatic cancer, bladder cancer, colorectal cancer, colon cancer, uterine/cervical cancer, prostate cancer, testicular cancer, esophageal cancer, gastrointestinal cancer, head and neck cancer, germ cell cancer, bone cancer, liver cancer, thyroid cancer, skin cancer, tumor of central nervous system, lymphoma, leukemia, myeloma, sarcoma, and melanoma.

In some embodiments, the infection is selected from the group consisting of viral infection, bacterial infection, fungal infection and parasitic infection.

In some embodiments, the subject is a mammal, such as a human.

The antibody or antigen-binding fragment thereof or single-arm antibody or pharmaceutical composition of the present invention can be formulated into any dosage form known in the medical field, for example, tablet, pill, suspension, emulsion, solution, gel, capsule, powder, granule, elixir, lozenge, suppository, injection (including injection solution, sterile powder for injection, and concentrated solution for injection), inhalant, spray, etc. The preferred dosage form depends on the intended mode of administration and therapeutic use. The antibody or antigen-binding fragment thereof or single-arm antibody or pharmaceutical composition of the present invention should be sterile and stable under production and storage conditions. A preferred dosage form is an injection. Such injection can be a sterile injection solution. For example, a sterile injection solution can be prepared by the following method: incorporating a necessary dose of the antibody or antigen-binding fragment thereof or single-arm antibody of the present invention into an appropriate solvent, and optionally, simultaneously incorporating an additional desired ingredient (including but not limited to, pH adjuster, surfactant, adjuvant, ionic strength enhancer, isotonic agent, preservative, diluent, or any combination thereof), followed by filtration for sterilization. In addition, the sterile injection solution can be prepared as a sterile lyophilized powder (e.g., by vacuum drying or freeze drying) for easy storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

The antibody or antigen-binding fragment thereof of the present invention, or the single-arm antibody of the present invention, or the pharmaceutical composition of the present invention can be administered by any suitable method known in the art, including but not limited to, oral, buccal, sublingual, ocular, topical, parenteral, rectal, intrathecal, intracytoplasmic, inguinal, intravesical, topical (e.g., powder, ointment or drops), or nasal route. However, for many therapeutic uses, the preferred route/mode of administration is parenteral administration (e.g., intravenous injection or bolus injection, subcutaneous injection, intraperitoneal injection, intramuscular injection). It should be understood by those skilled in the art that the route and/or mode of administration will vary depending on the intended purpose. In certain embodiments, the antibody or antigen-binding fragment thereof or the single-arm antibody or the pharmaceutical composition of the present invention is administered by intravenous injection or bolus injection.

### Detection use

In the tenth aspect, the present invention provides a conjugate, which comprises the antibody or antigen-binding fragment thereof of the first aspect or the single-arm antibody of the second aspect, and optionally a detectable label conjugated to the antibody or antigen-binding fragment thereof or the single-arm antibody.

In some embodiments, the detectable label is selected from the group consisting of enzyme (e.g., horseradish peroxidase or alkaline phosphatase), chemiluminescent reagent (e.g., acridinium ester compound, luminol and derivative thereof, or ruthenium derivative), a fluorescent dye (e.g., fluorescein or fluorescent protein), a radionuclide and biotin.

In the eleventh aspect, the present invention provides a kit, which comprises the antibody or antigen-binding fragment thereof of the first aspect or the single-arm antibody of the second aspect or the conjugate of the tenth aspect.

In some embodiments, the kit comprises the conjugate of the tenth aspect.

In some embodiments, the kit comprises the antibody or antigen-binding fragment thereof of the first aspect, and a second antibody capable of specifically recognizing the antibody or antigen-binding fragment thereof. In certain embodiments, the second antibody further comprises a detectable label, such as an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide or biotin.

In some embodiments, the kit comprises the single-arm antibody of the second aspect, and a second antibody capable of specifically recognizing the single-arm antibody. In certain embodiments, the second antibody further comprises a detectable label, such as an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide or biotin.

In the twelfth aspect, the present invention provides a method for detecting the presence or level of HER2 in a sample, which comprises using the antibody or antigen-binding fragment thereof of the first aspect or the single-arm antibody of the second aspect or the conjugate of the tenth aspect. In certain embodiments, the method is used for a therapeutic purpose or diagnostic purpose. In other embodiments, the method is used for a non-therapeutic and non-diagnostic purpose.

In some embodiments, the method is an immunological assay, such as immunoblotting, enzyme immunoassay (e.g., ELISA), chemiluminescent immunoassay, fluorescent immunoassay or radioimmunoassay.

In some embodiments, the method comprises using the conjugate of the tenth aspect.

In some embodiments, the method comprises using the antibody or antigen-binding fragment thereof of the first aspect, and the method further comprises using a second antibody carrying a detectable label (e.g., an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide or biotin) to detect the antibody or antigen-binding fragment thereof.

In some embodiments, the method comprises using the single-arm antibody of the second aspect, and the method further comprises using a second antibody carrying a detectable label (e.g., an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide or biotin) to detect the single-arm antibody.

In certain embodiments, the method comprises: (1) contacting the sample with the antibody or antigen-binding fragment thereof or the single-arm antibody of the present invention; (2) detecting the formation of an antigen-antibody immune complex or detecting the amount of the immune complex. The formation of the immune complex indicates the presence of HER2 or a cell expressing HER2.

In the thirteenth aspect, the present invention provides the use of the antibody or antigen-binding fragment thereof of the first aspect or the single-arm antibody of the second aspect or the conjugate of the tenth aspect in the manufacture of a detection reagent for detecting the presence or level of HER2 in a sample.

In some embodiments, the detection reagent detects the presence or level of HER2 in a sample by the method for detecting the presence or level of HER2 in a sample of the twelfth aspect.

In some embodiments, the sample is a cell sample (e.g., an immune cell) from a subject (e.g., a mammal, preferably a human or a cynomolgus monkey).

### Definition of terms

In the present invention, unless otherwise specified, the scientific and technical terms used herein have the meanings commonly understood by those skilled in the art. In addition, the virology, biochemistry, and immunology laboratory operation steps used herein are all routine steps widely used in the corresponding fields. At the same time, in order to better understand the present invention, the definitions and explanations of relevant terms are provided below.

When the terms "for example," "such as," "e.g.," "including," "comprising," or variants thereof are used herein, these terms will not be considered as restrictive terms, but will be interpreted as meaning "but not being limited to" or "not being limited to."

Unless otherwise specified herein or clearly contradicted by the context, the terms "a" and "an" as well as "the" and similar designations should be interpreted as covering the singular and plural in the context of describing the present invention (especially in the context of the following claims).

The term "antibody" used herein refers to an immunoglobulin-derived molecule that is capable of specifically binding to a target antigen, and the immunoglobulin-derived molecule binds to the target antigen through at least one antigen binding site located in its variable region. When referring to the term "antibody", unless the context clearly indicates otherwise, it includes not only intact antibody, but also antigen-binding fragments that are capable of specifically binding to the target antigen. "Intact antibody" is typically composed of two pairs of polypeptide chains, each pair having one light chain (LC) and one heavy chain (HC). Antibody light chains can be classified as κ (kappa) and λ (lambda) light chains. Heavy chains can be classified as µ, δ, γ, α or ε, and define antibody isotypes as IgM, IgD, IgG, IgA and IgE, respectively. Within the light and heavy chains, the variable and constant regions are connected by a "J" region of about 12 or more amino acids, and the heavy chain also contains a "D" region of about 3 or more amino acids. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain, CL. The constant domain is not directly involved in the binding of antibodies to antigens, but exhibits a variety of effector functions, such as mediating the binding of immunoglobulins to host tissues or factors, including various cells (e.g., effector cells) of the immune system and the first component (C1q) of the classical complement system. The VH and VL regions can also be subdivided into regions with high variability (called complementarity determining regions (CDRs)), interspersed with more conservative regions called framework regions (FRs). Each V_{H} and V_{L} consists of three CDRs and four FRs arranged from the amino terminal to the carboxyl terminal in the following order: FR1, CDR1, FR2, CDR2, FR3, CDR3, FR4. The variable regions (VH and VL) of each heavy chain/light chain pair form antigen binding sites, respectively. The distribution of amino acids in various regions or domains can follow the definition of Kabat, Sequences of Proteins of Immunological Interest (National Institutes of Health, Bethesda, Md. (1987 and 1991)), or Chothia & Lesk (1987) J. Mol. Biol. 196: 901-917; Chothia et al. (1989) Nature 342: 878-883.

As used herein, the term "complementarity determining region" or "CDR" refers to the amino acid residues in variable region of an antibody that are responsible for antigen binding. There are three CDRs in each of the heavy and light chain variable regions, designated as CDR1, CDR2, and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196: 901-917; Chothia et al. (1989) Nature 342: 878-883), or the IMGT numbering system (Lefranc et al., Dev. Comparat. Immunol. 27: 55-77, 2003). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27: 55-77, 2003).

As used herein, the term "framework region" or "FR" residues refers to those amino acid residues in a variable region of an antibody other than the CDR residues defined above.

The term "antibody" is not limited to any particular method for antibody production. For example, it includes recombinant antibodies, monoclonal antibodies and polyclonal antibodies. The antibody can be an antibody of different isotypes, for example, IgG (e.g., IgG1, IgG2, IgG3 or IgG4 subtypes), IgA1, IgA2, IgD, IgE or IgM antibody.

As used herein, the term "antigen-binding fragment" of antibody refers to a polypeptide comprising a fragment of a full-length antibody that retains the ability to specifically bind to the same antigen bound by the full-length antibody and/or competes with the full-length antibody for specific binding to the antigen, which is also referred to as an "antigen-binding portion". See generally, Fundamental Immunology, Ch. 7 (Paul, W., ed., 2nd ed., Raven Press, N.Y. (1989), which is incorporated herein by reference in its entirety for all purposes. Antigen-binding fragments of antibodies can be produced by recombinant DNA techniques or by enzymatic or chemical cleavage of intact antibodies. Non-limiting examples of antigen-binding fragments include Fab, Fab', F(ab')₂, Fd, Fv, complementarity determining region (CDR) fragments, scFv, diabody, single domain antibody, chimeric antibody, linear antibody, nanobody (technology from Domantis), probody, and polypeptides that contain at least a portion of antibody sufficient to confer specific antigen binding ability to the polypeptides. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains". Wherein, "full-length heavy chain" refers to a polypeptide chain that is composed of a heavy chain variable region (VH), a heavy chain constant region CH1 domain, a hinge region (HR), a heavy chain constant region CH2 domain, and a heavy chain constant region CH3 domain in the direction from N-terminal to C-terminal; and, when the full-length antibody is an IgE isotype, it optionally also comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. Two pairs of full-length antibody chains are linked together by disulfide bonds between CL and CH1 and between the HRs of the two full-length heavy chains. The full-length antibody of the present invention may be derived from a single species, such as human; and it may also be a chimeric antibody or humanized antibody. The full-length antibody of the present invention comprises two antigen binding sites formed by VH and VL pairs, respectively, which specifically recognize/bind to the same antigen.

As used herein, the term "Fd" refers to an antibody fragment consisting of VH and CH1 domains; the term "dAb fragment" refers to an antibody fragment consisting of a VH domain (Ward et al., Nature 341: 544-546 (1989)); the term "Fab fragment" refers to an antibody fragment consisting of VL, VH, CL and CH1 domains; the term "F(ab')₂ fragment" refers to an antibody fragment comprising two Fab fragments linked by a disulfide bridge at the hinge region; the term "Fab' fragment" refers to a fragment obtained after reduction of the disulfide bonds linking the two heavy chain fragments in F(ab')₂ fragment, consisting of a complete light chain and the Fd fragment of heavy chain (consisting of VH and CH1 domains).

As used herein, the term "Fv" refers to an antibody fragment consisting of the VL and VH domains of a single arm of an antibody. The Fv fragment is generally considered to be the smallest antibody fragment that can form a complete antigen binding site. It is generally believed that the six CDRs confer antigen-binding specificity to an antibody. However, even one single variable region (e.g., Fd fragment, which contains only three CDRs specific for an antigen) can recognize and bind to the antigen, although its affinity may be lower than that of the complete binding site.

As used herein, the term "scFv" refers to a single polypeptide chain comprising VL and VH domains, wherein the VL and VH are connected by a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, Vol. 113, Roseburg and Moore, ed., Springer-Verlag, New York, pp. 269-315 (1994)). Such scFv molecules may have a general structure: NH₂-VL-linker-VH-COOH or NH₂-VH-linker-VL-COOH. Suitable linkers in the prior art consist of repeated GGGGS amino acid sequences or variants thereof. For example, a linker having amino acid sequence (GGGGS)₄ can be used, but variants thereof can also be used (Holliger et al. (1993), Proc. Natl. Acad. Sci. USA 90: 6444-6448). Other linkers that can be used in the present invention are described by Alfthan et al. (1995), Protein Eng. 8: 725-731, Choi et al. (2001), Eur. J. Immunol. 31: 94-106, Hu et al. (1996), Cancer Res. 56: 3055-3061, Kipriyanov et al. (1999), J. Mol. Biol. 293: 41-56, and Roovers et al. (2001), Cancer Immunol. In some cases, a disulfide bond may also exist between the VH and VL of the scFv. In certain embodiments of the present invention, scFv may form a di-scFv, which refers to an antibody formed by connecting two or more single scFvs in series. In certain embodiments of the present invention, scFv may form a (scFv)₂, which refers to an antibody formed by connecting two or more single scFvs in parallel.

As used herein, the term "diabody" means that its VH and VL domains are expressed on a single polypeptide chain, but a linker that is too short to allow pairing between the two domains of the same chain is used, thereby forcing the domains to pair with the complementary domains of another chain and generate two antigen binding sites (see, for example, Holliger P. et al., Proc. Natl. Acad. Sci. USA 90: 6444-6448 (1993), and Poljak R. J. et al., Structure 2: 1121-1123 (1994)).

As used herein, the term "single-domain antibody (sdAb)" has the meaning generally understood by those skilled in the art, which refers to an antibody fragment composed of a single monomeric variable antibody domain (e.g., a single heavy chain variable region), and retains the ability to specifically bind to the same antigen bound by a full-length antibody. Single-domain antibody is also called nanobody.

Each of the above antibody fragments retains the ability to specifically bind to the same antigen bound by a full-length antibody, and/or competes with the full-length antibody for specific binding to the antigen.

Antibody antigen-binding fragments (e.g., the above antibody fragments) can be obtained from a given antibody (e.g., the antibody provided by the present invention) using conventional techniques known to those skilled in the art (e.g., recombinant DNA technology or enzymatic or chemical cleavage methods), and the antigen-binding fragments of antibody can be screened for specificity in the same manner as for the intact antibody.

As used herein, the term "chimeric antibody" refers to an antibody in which a portion of light chain or/and heavy chain is derived from one antibody (which may be derived from a particular species or belong to a particular antibody class or subclass), and another portion of light chain or/and heavy chain is derived from another antibody (which may be derived from the same or different species or belong to the same or different antibody class or subclass), but in any case, it still retains binding activity to target antigen (U.S.P 4,816,567 to Cabilly et al.; Morrison et al., Proc. Natl. Acad. Sci. USA, 81: 6851-6855 (1984)). In certain embodiments, the term "chimeric antibody" may include an antibody in which the heavy chain and light chain variable regions of the antibody are derived from a first antibody, and the heavy chain and light chain constant regions of the antibody are derived from a second antibody.

As used herein, the term "Fc domain" or "Fc region" refers to a portion of heavy chain constant region containing CH2 and CH3. The Fc fragment of an antibody has a variety of different functions, but does not participate in antigen binding. "Effector functions" mediated by the Fc region include Fc receptor binding; Clq binding and complement-dependent cytotoxicity (CDC); antibody-dependent cell-mediated cytotoxicity (ADCC); phagocytosis; downregulation of cell surface receptors (e.g., B cell receptor); and B cell activation, among others. The Fc region can be any antibody heavy chain constant region isotype, such as IgG1, IgG2, IgG3, or IgG4.

The Fc domain can include both native Fc regions and variant Fc regions. A native Fc region comprises an amino acid sequence that is consistent with the amino acid sequence of an Fc region found in nature, For example, a native sequence human Fc region includes a native sequence human IgG1 Fc region (non-A and A allotypes); a native sequence human IgG2 Fc region; a native sequence human IgG3 Fc region; and a native sequence human IgG4 Fc region, as well as naturally occurring variants thereof. A variant Fc region comprises an amino acid sequence that differs from the amino acid sequence of a native sequence Fc region due to at least one amino acid modification. In some embodiments, the variant Fc region may have an effector function (e.g., Fc receptor binding, antibody glycosylation, number of cysteine residues, effector cell function, or complement function) that is altered compared to the native Fc region. In some embodiments, the variant Fc region may have a modification that promotes dimerization.

As used herein, the term "identity" is used to refer to the matching of sequences between two polypeptides or between two nucleic acids. To determine the percent identity of two amino acid sequences or two nucleic acid sequences, the sequences are aligned for optimal comparison purposes (e.g., a gap may be introduced in a first amino acid sequence or nucleic acid sequence to optimally align with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between two sequences is a function of the number of identical positions shared by the sequences (i.e., percent identity = number of identical overlapping positions/total number of positions × 100%). In certain embodiments, the two sequences are of the same length.

The determination of the percent identity between two sequences can also be achieved using a mathematical algorithm. A non-limiting example of the mathematical algorithm for comparison of two sequences is the algorithm of Karlin and Altschul, 1990, Proc. Natl. Acad. Sci. U.S.A. 87: 2264-2268, as modified in Karlin and Altschul, 1993, Proc. Natl. Acad. Sci. U.S.A. 90: 5873-5877. Such an algorithm is incorporated into the NBLAST and XBLAST programs of Altschul et al., 1990, J. Mol. Biol. 215: 403.

As used herein, the term "variant", in the context of a polypeptide (including a polypeptide), also refers to a polypeptide or peptide comprising an amino acid sequence that has been altered by introducing a substitution, deletion or addition of amino acid residues. In some cases, the term "variant" also refers to a polypeptide or peptide that has been modified (i.e., by covalently connecting any type of molecule to the polypeptide or peptide). For example, but not limiting, the polypeptide can be modified, for example, by glycosylation, acetylation, pegylation, phosphorylation, amidation, derivatization by known protecting/blocking groups, proteolytic cleavage, connection to cellular ligands or other proteins, etc. Derivatized polypeptides or peptides can be produced by chemical modification using techniques known to those skilled in the art, and the techniques include but are not limited to specific chemical cleavage, acetylation, formylation, metabolic synthesis of tunicamycin, etc. In addition, the variant has similar, identical or improved functions to the polypeptide or peptide from which it is derived.

As used herein, the term "specific binding" refers to a non-random binding reaction between two molecules, such as a reaction between an antibody and an antigen to which it is directed. The strength or affinity of a specific binding interaction can be expressed by the equilibrium dissociation constant (K_{D}) of the interaction. In the present invention, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding and the higher the affinity between the antibody and the antigen.

The specific binding properties between two molecules can be determined using methods known in the art. One method involves measuring the rate of association and dissociation of antigen binding site/antigen complexes. Both the "association rate constant" (ka or kon) and the "dissociation rate constant" (kdis or koff) can be calculated from concentrations and the actual rates of association and dissociation (see, Malmqvist M, Nature, 1993, 361: 186-187). The ratio of kdis/kon is equal to the dissociation constant K_{D} (see, Davies et al., Annual Rev Biochem, 1990; 59: 439-473). KD, kon and kdis values can be measured by any effective method. In certain embodiments, the dissociation constant can be measured in Biacore using surface plasmon resonance (SPR). In addition, the dissociation constant can be measured by bioluminescence interferometry or Kinexa.

As used herein, the detectable label of the present invention can be any substance that can be detected by means of fluorescence, spectroscopy, photochemistry, biochemistry, immunology, electrical, optical or chemistry. Such labels are well known in the art, and examples thereof include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C or ³²P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750)), luminescent substances (e.g., chemiluminescent substances, such as acridinium ester compounds, luminol and derivatives thereof, ruthenium derivatives such as terpyridine ruthenium), magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above labels.

As used herein, the term "vector" refers to a nucleic acid carrier into which a polynucleotide can be inserted. When a vector can express the protein encoded by the inserted polynucleotide, the vector is called an expression vector. The vector can be introduced into a host cell by transformation, transduction or transfection, so that the genetic material elements it carries are expressed in the host cell. Vectors are well known to those skilled in the art, and include but are not limited to: plasmid; phagemid; cosmid; artificial chromosome such as yeast artificial chromosome (YAC), bacterial artificial chromosome (BAC) or P1-derived artificial chromosome (PAC); bacteriophage such as λ phage or M13 phage, and animal viruses, etc. Animal viruses that can be used as vectors include but are not limited to retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including but not limited to promoter sequence, transcription initiation sequence, enhancer sequence, selection element and reporter gene. In addition, the vector may also contain a replication origin site.

As used herein, the term "host cell" refers to a cell that can be used to introduce a vector, including but not limited to prokaryotic cell such as *Escherichia coli* or *Bacillus subtilis,* fungal cell such as yeast cell or *Aspergillus,* insect cell such as *S2 Drosophila* cell or *Sf9*, or animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or change the expected properties of the protein/polypeptide comprising the amino acid sequence. For example, conservative substitution can be introduced by standard techniques known in the art, such as site-directed mutagenesis and PCR-mediated mutagenesis. Conservative amino acid substitutions include substitutions of amino acid residues with amino acid residues having similar side chains, such as substitutions with residues that are physically or functionally similar to the corresponding amino acid residues (e.g., having similar size, shape, charge, chemical properties, including the ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative substitutions of amino acids are well known in the art (see, for example, Brummell et al., Biochem. 32: 1180-1187 (1993); Kobayashi et al. Protein Eng. 12(10): 879-884 (1999); and Burks et al. Proc. Natl Acad. Set USA 94: 412-417 (1997), which are incorporated herein by reference).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutically acceptable carriers and/or excipients" refers to carriers and/or excipients that are pharmacologically and/or physiologically compatible with a subject and an active ingredient, which is well known in the art (see, for example, Remington's Pharmaceutical Sciences. Edited by Gennaro AR, 19th ed. Pennsylvania: Mack Publishing Company, 1995), and include, but are not limited to: pH regulator, surfactant, adjuvant, ionic strength enhancer, diluent, agent for maintaining osmotic pressure, agent for delaying absorption, preservative. For example, the pH regulator includes, but is not limited to, phosphate buffer. The surfactant includes, but is not limited to, cationic, anionic or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, and the like. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), etc. The preservative includes, but is not limited to, various antibacterial and antifungal agents, such as thimerosal, 2-phenoxyethanol, paraben, chlorobutanol, phenol, sorbic acid, etc. The stabilizer has the meaning generally understood by those skilled in the art, which can stabilize the desired activity of an active ingredient in drug, and includes, but is not limited to, sodium glutamate, gelatin, SPGA, sugar (e.g., sorbitol, mannitol, starch, sucrose, lactose, dextran, or glucose), amino acid (e.g., glutamic acid, glycine), protein (e.g., dried whey, albumin or casein) or degradation product thereof (e.g., lactalbumin hydrolysate), etc. In certain exemplary embodiments, the pharmaceutically acceptable carriers or excipients include a sterile injectable liquid (e.g., aqueous or non-aqueous suspensions or solution). In certain exemplary embodiments, such sterile injectable liquids are selected from water for injection (WFI), bacteriostatic water for injection (BWFI), sodium chloride solution (e.g., 0.9% (w/v) NaCl), glucose solution (e.g., 5% glucose), solution containing surfactant (e.g., 0.01% polysorbate 20), pH buffer solution (e.g., phosphate buffer solution), Ringer's solution, and any combination thereof.

As used herein, the term "prevention" refers to a method implemented to prevent or delay the occurrence of a disease or disorder or symptom in a subject. As used herein, the term "treatment" refers to a method implemented to obtain a beneficial or desired clinical result. For the purposes of the present invention, beneficial or desired clinical results include (but are not limited to) alleviation of symptom, reduction of disease extent, stabilization (i.e., no longer worsening) of disease state, delay or slowing disease development, improvement or alleviation of disease state, and relief of symptom (whether partial or complete), whether detectable or undetectable. In addition, "treatment" may also refer to prolonging survival compared to the expected survival if not receiving treatment.

As used herein, the term "subject" refers to a mammal, such as a human. In certain embodiments, the subject (e.g., human) suffers from a tumor, infection, or autoimmune disease, or is at risk of suffering from the above diseases.

As used herein, the term "effective amount" refers to an amount sufficient to obtain or at least partially obtain the desired effect. For example, an effective amount for preventing a disease (e.g., a tumor, infection, or autoimmune disease) refers to an amount sufficient to prevent, arrest, or delay the occurrence of the disease; an effective amount for treating a disease refers to an amount sufficient to cure or at least partially prevent the disease and its complications in a patient who already has the disease. Determining such an effective amount is well within the capabilities of those skilled in the art. For example, an effective amount for therapeutic use will depend on the severity of the disease to be treated, the overall state of the patient's own immune system, the patient's general condition such as age, weight, and sex, the mode of administration of drug, and other treatments administered simultaneously, etc.

### Beneficial effects of the present invention

The present invention provides anti-HER2 antibodies, which have a high binding affinity with HER2, cross-reactivity with human and cynomolgus monkey HER2, good thermal stability, and excellent anti-tumor effects in various tumors (e.g., neurocytoma, breast cancer, ovarian cancer, gastric cancer, lung cancer, kidney cancer, intestinal cancer, pancreatic cancer, bladder cancer, etc.).

### Brief Description of the Drawings

Figure 1 shows a structural schematic diagram of the monoclonal antibody and single-arm antibody of the present invention.
Figure 2A and Figure 2B show the binding affinity of the monoclonal antibody of the present invention with HER2 protein.
Figure 3 shows the detection results of binding of the monoclonal antibody of the present invention to human HER2 on N87 cells overexpressing HER2.
Figure 4 shows the detection results of blocking of HER2 signal-dependent cell proliferation by the monoclonal antibody of the present invention on N87 cells overexpressing HER2.
Figure 5 shows the ADCC effect of the monoclonal antibody of the present invention mediated by HER2.
Figure 6 shows the charge heterogeneity analysis of the monoclonal antibody of the present invention after high-temperature treatment, wherein 0H represents the sample before high-temperature treatment; HT2W represents the sample after high-temperature treatment for 2 weeks; HT4W represents the sample after high-temperature treatment for 4 weeks.
Figure 7 shows the activity verification of the monoclonal antibody of the present invention after high-temperature treatment, wherein 0H represents the sample before high-temperature treatment; HT2W represents the sample after high-temperature treatment for 2 weeks; HT4W represents the sample after high-temperature treatment for 4 weeks.

The embodiments of the present invention will be described in detail below in conjunction with the accompanying drawings and examples, but those skilled in the art will understand that the following drawings and examples are only used to illustrate the present invention, rather than to limit the scope of the present invention. According to the following detailed description of the accompanying drawings and preferred examples, the various objects and advantages of the present invention will become apparent to those skilled in the art.

### Sequence information

Post-translational modification sites (PTM) may have negative influences on the stability and safety of drugs and reduce the efficacy of drugs. In the present invention, Trastuzumab sequence was optimized to remove its post-translational modification sites (PTM), and underwent random mutation of the antibody heavy and light chain CDR regions to construct an antibody library, and then high-affinity antibodies were screened out from the antibody library by yeast display technology. In the present invention, the amino acid sequences encoding the antibodies were specifically disclosed, and the relevant data of the candidate antibodies were further shown.

The description of the sequences involved in the present invention is provided in the following Table 1-1 and Table 1-2.

**Table 1-1. Summary of sequences of variable regions and CDRs of the present invention**

| Sequence name | Sequence No. | CDR1 | CDR2 | CDR3 |
|---|---|---|---|---|
| Trastuzumab-HC | SEQ ID NO: 1 | SEQ ID NO: 2 | SEQ ID NO: 3 | SEQ ID NO: 4 |
| Trastuzumab-LC | SEQ ID NO: 5 | SEQ ID NO: 6 | SEQ ID NO: 7 | SEQ ID NO: 8 |
| Trastuzumab-PTM removal-HC | SEQ ID NO: 9 | SEQ ID NO: 10 | SEQ ID NO: 11 | SEQ ID NO: 12 |
| Trastuzumab-PTM removal-LC | SEQ ID NO: 13 | SEQ ID NO: 14 | SEQ ID NO: 15 | SEQ ID NO: 16 |
| Anti-HER2(NO3-02)-HC | SEQ ID NO: 9 | SEQ ID NO: 10 | SEQ ID NO: 11 | SEQ ID NO: 12 |
| Anti-HER2(NO3-02)-LC | SEQ ID NO: 17 | SEQ ID NO: 18 | SEQ ID NO: 19 | SEQ ID NO: 20 |
| Anti-HER2(NO3-36)-HC | SEQ ID NO: 9 | SEQ ID NO: 10 | SEQ ID NO: 11 | SEQ ID NO: 12 |
| Anti-HER2(NO3-36)-LC | SEQ ID NO: 21 | SEQ ID NO: 22 | SEQ ID NO: 23 | SEQ ID NO: 24 |
| Anti-HER2(NO3-46)-HC | SEQ ID NO: 9 | SEQ ID NO: 10 | SEQ ID NO: 11 | SEQ ID NO: 12 |
| Anti-HER2(NO3-46)-LC | SEQ ID NO: 25 | SEQ ID NO: 26 | SEQ ID NO: 27 | SEQ ID NO: 28 |

**Table 1-2. Summary of sequences of constant regions and other sequences of the present invention**

| Sequence name | Sequence No. |
|---|---|
| Human IgG1 constant region | SEQ ID NO: 29 |
| Human IgG1 constant region with Knob mutation | SEQ ID NO: 30 |
| Human Kappa constant region | SEQ ID NO: 31 |
| Human Fc region with Hole mutation | SEQ ID NO: 32 |
| X₁X₂VQX₃A | SEQ ID NO: 33 |
| QQHX₄X₅TPPT | SEQ ID NO: 34 |

SEQ ID NO: 1
SEQ ID NO: 2
   GFNIKDTY
SEQ ID NO: 3
   IYPTNGYT
SEQ ID NO: 4
   SRWGGDGFYAMDY
SEQ ID NO: 5
SEQ ID NO: 6
   QDVNTA
SEQ ID NO: 7
   SAS
SEQ ID NO: 8
   QQHYTTPPT
SEQ ID NO: 9 
EVQLVESGGGLVQPGGSLRLSCAASGFNIKDTYIHWVRQAPGKGLEWVARIYPTQ
GYTRYADSVKGRFTISADTSKNTAYLQMNSLRAEDTAVYYCSRWGGEGFYAMDYWG
QGTLVTVSS
SEQ ID NO: 10
   GFNIKDTY
SEQ ID NO: 11
   IYPTQGYT
SEQ ID NO: 12
   SRWGGEGFYAMDY
SEQ ID NO: 13 
DIQMTQSPSSLSASVGDRVTITCRASQDVQTAVAWYQQKPGKAPKLLIYSASFLYS
GVPSRFSGSRSGTDFTLTISSLQPEDFATYYCQQHYTTPPTFGQGTKVEIK
SEQ ID NO: 14
   QDVQTA
SEQ ID NO: 15
   SAS
SEQ ID NO: 16
   QQHYTTPPT
SEQ ID NO: 17 
DIQMTQSPSSLSASVGDRVTITCRASQNVQGAVAWYQQKPGKAPKLLIYSASFLYS
GVPSRFSGSRSGTDFTLTISSLQPEDFATYYCQQHYSTPPTFGQGTKVEIK
SEQ ID NO: 18
   QNVQGA
SEQ ID NO: 19
   SAS
SEQ ID NO: 20
   QQHYSTPPT
SEQ ID NO: 21 
DIQMTQSPSSLSASVGDRVTITCRASNYVQTAVAWYQQKPGKAPKLLIYSASFLYS
GVPSRFSGSRSGTDFTLTISSLQPEDFATYYCQQHFMTPPTFGQGTKVEIK
SEQ ID NO: 22
   NYVQTA
SEQ ID NO: 23
   SAS
SEQ ID NO: 24
   QQHFMTPPT
SEQ ID NO: 25 
DIQMTQSPSSLSASVGDRVTITCRASQSVQGAVAWYQQKPGKAPKLLIYSASFLYS
GVPSRFSGSRSGTDFTLTISSLQPEDFATYYCQQHSTTPPTFGQGTKVEIK
SEQ ID NO: 26
   QSVQGA
SEQ ID NO: 27
   SAS
SEQ ID NO: 28
   QQHSTTPPT
SEQ ID NO: 29 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPG
SEQ ID NO: 30 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
 
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPCRDELTKNQVSLWCLVKGFYPSDIAVEWESNGQPENNYKTTPPVLDSDGSFFLYSK
LTVDKSRWQQGNVFSCSVMHEALHNHYTQKSLSLSPG
SEQ ID NO: 31
SEQ ID NO: 32

DKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEK
TISKAKGQPREPQVCTLPPSRDELTKNQVSLSCAVKGFYPSDIAVEWESNGQPENNYKTT
PPVLDSDGSFFLVSKLTVDKSRWQQGNVFSCSVMHEALHNHYTQKSLSLSPG

### Specific Models for Carrying Out the present invention

The present invention is now described with reference to the following examples which are intended to illustrate the present invention (but not to limit the present invention).

Unless otherwise specified, the molecular biology experimental methods and immunoassays used in the present invention are performed basically based on the methods described in J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd edition, John Wiley & Sons, Inc., 1995; restriction endonucleases were used in accordance with the conditions recommended by the product manufacturer. It is known to those skilled in the art that the examples describe the present invention by way of example and are not intended to limit the scope of protection of the present invention.

### Example 1: Construction and screening of monoclonal antibody affinity maturation library

### 1.1. Construction of affinity maturation library

Trastuzumab heavy chain variable region gene fragment (amino acid sequence was set forth in SEQ ID NO: 1), light chain variable region gene fragment (amino acid sequence was set forth in SEQ ID NO: 5), heavy chain variable region gene fragment introduced with N62Q and D110E mutations (amino acid sequence was set forth in SEQ ID NO: 9), and light chain variable region gene fragment introduced with N36Q mutation (amino acid sequence was set forth in SEQ ID NO: 13), were synthesized. VH and VK with removal of PTM were used as templates, degenerate primers were designed for CDR2 and CDR3 of VH and CDR3 of VK, respectively. To ensure that each amino acid could be mutated into any of the 20 amino acids, PCR amplification was performed using NNK as the form of mutagenic bases. The target fragments were recovered using a PCR purification kit (purchased from QIAGEN). The linearized yeast display vector and the PCR products of VH and VK were mixed and electrotransformed into *Saccharomyces cerevisiae* to construct affinity maturation libraries of heavy chain mutations and light chain mutations, respectively, and their library capacities were determined.

### 1.2. Screening of anti-HER2 antibodies

### 1.2.1 Biotinylation labeling of HER2 protein

An appropriate volume of double distilled water was taken to dissolve human HER2 protein (purchased from AcroBiosystems). According to the product instructions of biotin labeling kit (purchased from Thermo), biotin was dissolved and mixed with the protein solution, and incubated at 4°C for 2 hours. A desalting column (purchased from Thermo) was used to remove excess biotin, in which the pretreatment steps for the desalting column and sample collection were all performed according to the steps in the product instructions.

### 1.2.2 MACS enrichment of yeast cells capable of specifically binding to human HER2

The antibody libraries constructed in Example 1.1 were inoculated into SD-CAA amplification medium (1L of SD-CAA amplification medium contained 6.7g of YNB, 5g of casamino acids, 13.62g of Na₂HPO₄·12H₂O, 7.44g of NaH₂PO₄ and 2% glucose), and cultured overnight at 30°C and 225 rpm. The yeast cells were taken at an appropriate amount, and centrifuged at 3000rpm×5min (the following centrifugation operation was the same) to remove the medium. The yeast cells were resuspended with SD-CAA induction medium, and induced overnight. After the induction, the concentrations of the libraries were determined. The yeast cells were taken at an appropriate amount, and centrifuged to remove the medium. The yeast cells were resuspended with 50ml of PBS, and centrifuged to remove the supernatant. The yeast cells were resuspended with 10ml of PBS.

The biotin-labeled human HER2 protein (final concentration of 100nM) was added, incubated at room temperature for 30min. The yeast cells were collected by centrifugation, and washed 3 times with 50mL of PBS. The yeast cells were resuspended with 5ml of washing solution, added with 200µl of SA magnetic beads (purchased from Miltenyi Biotec), and incubated upside down for 10min. The mixture of yeast cells and magnetic beads were washed 3 times with PBS, and then the mixture was added to a LS purification column (purchased from Miltenyi Biotec). The LS purification column was placed on the magnetic grate and the non-specifically bound yeast cells were removed by washing with PBS. The purification column was taken from the magnetic grate, and the yeast cells were eluted by adding PBS. The eluted yeast cells were centrifuged, then transferred into a SD-CAA amplification medium for amplification.

### 1.2.3 Flow cytometric sorting to obtain high-affinity yeast cells

The yeast cells enriched by MACS were inoculated into SD-CAA amplification medium, and cultured in a shake flask overnight at 30°C and 225rpm. The yeast cells were resuspended with SD-CAA induction medium and induced overnight. Anti-c-Myc mouse antibody (purchased from Thermo) and 100nM biotin-labeled HER2 antigen were added, and incubated for 10 minutes. The yeast cells were washed 3 times by adding PBS, added with goat anti-mouse IgG (H+L) Alexa Fluor Plus 488 fluorescent antibody (purchased from Invitrogen) and streptavidin APC conjugate fluorescent antibody (purchased from Invitrogen), and incubated for 15 minutes. The cells were resuspended by adding PBS, and sorted by a BD FACSAria III instrument to obtain yeast cells with high binding ability to HER2 antigen.

### 1.2.4 Retrieval of antibody gene of HER2 antibody candidate molecule

The yeast liquid with high binding ability to human HER2 antigen obtained by MACS and FACS enrichment was coated on a solid culture plate of SD-CAA, and then single clones were picked and cultured overnight at 30°C and 225rpm in SD-CAA amplification medium. The amplified single clones were treated with 0.1% SDS, centrifuged, and the supernatant as a template underwent PCR amplification. The PCR product was sequenced to obtain the gene sequence.

### Example 2: Construction, expression and purification of monoclonal antibodies

### 2.1. Construction of monoclonal antibody gene into pCDNA3.1 expression vector

The heavy chain variable region gene sequence was linked to the human IgG1 constant region gene sequence (amino acid sequence was set forth in SEQ ID NO: 29) and constructed into the EcoR I/Not I double-digested linearized pCDNA3.1 vector using homologous recombinase (purchased from Vazyme); the light chain variable region gene sequence was linked to the human Kappa constant region gene sequence (amino acid sequence was set forth in SEQ ID NO: 31) and constructed into the EcoR I/Xhol I double-digested linearized pCDNA3.1 vector. The process was in accordance with the product instructions. The homologous recombination product was transferred into Top10 competent cells, the cells were coated on an ampicillin-resistant plate and cultured overnight at 37°C, single clones were picked for sequencing, and plasmids were extracted.

### 2.2. Cell transfection and protein purification

HEK293 cells were used for transfection and expression. The cell density was adjusted to 2.5×10⁶ cells/ml one day before transfection. On the next day, the cells were diluted to 3.0×10⁶ cells/ml for transfection. MEM medium was used as transfection buffer. PEI was added at a ratio of PEI: plasmid = 3: 1. The extracted heavy chain and light chain plasmids were co-transfected into HEK293 cells. After 5 days of cell culture, the supernatant was collected and the target protein was purified using a Protein A packing column. 10 times column volume of PBS was added to the packing column to balance the packing column, the above cell supernatant was added to the gravity column, and flowed through under gravity. After the sample was loaded, 20 times column volume of PBS was added to wash away the unbound impurities. When no liquid flowed out, the column was placed on a collection tube with neutralization buffer (1M Tris, pH8.54) added in advance, and 3-5 times column volume of elution buffer (0.1M sodium citrate, pH3.2) was used to elute the target protein.

### Example 3: Construction, expression and purification of Anti-HER2 single-arm antibody

To further confirm the affinity of the monovalent antibody, the heavy chain variable region gene sequence was linked to the Knob mutation-containing human IgG1 constant region gene sequence (amino acid sequence was set forth in SEQ ID NO: 30), and constructed into the EcoR I/Not I double-digested linearized pCDNA3.1 vector using homologous recombinase (purchased from Vazyme); the light chain variable region gene sequence was linked to the human Kappa light chain constant region gene sequence (amino acid sequence was set forth in SEQ ID NO: 31), and constructed into the EcoR I/Xhol I double-digested linearized pCDNA3.1 vector, and the gene sequence encoding human Fc region with Hole mutation (amino acid sequence was set forth in SEQ ID NO: 32) was constructed into the EcoRI/XhoI double-digested linearized pCDNA3.1 vector. The process was in accordance with the product instructions. The homologous recombination product was transferred into Top10 competent cells, the cells were coated on an ampicillin-resistant plates and cultured at 37°C overnight, the single clones were picked for sequencing, and plasmids were extracted. Cell transfection and protein purification were performed according to Example 2.2.

### Example 4: Purity measurement of Anti-HER2 antibody

In this study, HPLC was used to detect the purity of the protein. The HPLC method was as follows: mobile phase: 150mM Na₂HPO₄·12H₂O, pH7.0. Chromatographic conditions: detection wavelength: 280 nm, column temperature: 25°C, flow rate: 0.35 mL/min, detection time: 20 min, Zenix-C SEC-300 column (SEPAX 4.6×300mm, 3µm).

The results were shown in Table 2. The antibody molecules of the present invention after affinity purification had good purity and met the requirements of downstream process development.

**Table 2. Purity measurement results of Anti-HER2 antibody**

| No. | Monomer ratio (%) |
|---|---|
| Trastuzumab | 97.6% |
| Trastuzumab-PTM removal | 98.1% |
| Anti-HER2(NO3-02) | 94.6% |
| Anti-HER2(NO3-36) | 94.6% |
| Anti-HER2(NO3-46) | 96.4% |
| Trastuzumab_single arm | 90.9% |
| Trastuzumab-PTM removal_single arm | 95.2% |
| Anti-HER2(NO3-02)_single arm | 95.8% |
| Anti-HER2(NO3-36)_single arm | 95.9% |
| Anti-HER2(NO3-46)_single arm | 95.1% |

### Example 5: Thermal stability of Anti-HER2 monoclonal antibody

The thermal stability of different antibodies was measured by DSC (Differential scanning calorimetry). The sample was concentrated and diluted to 1 mg/mL with PBS; 5000× fluorescent chromogenic agent Sypro Orange (purchased from Bio-Rad) was diluted 50 times with ultrapure water to obtain 100× fluorescent chromogenic agent Sypro Orange. 50µL of 1mg/mL sample was taken, added with 10µL of 100× fluorescent chromogenic agent Sypro Orange and 40µL of ultrapure water, and mixed well, then 30µL of the mixture was taken and added to a 96-well PCR plate, 3 replicates for each sample, and placed in a PCR instrument, in which the heating program was set as: 25°C constant temperature for 5min, and heating to 99°C at a rate of 0.5°C/min. After the program was completed, the temperature value of the lowest point of the curve in the "Melt Curve" graph was read, which was the Tm value of the sample. The specific results were shown in Table 3 below, which indicated that point mutations and affinity optimization did not reduce the thermal stability of antibodies.

**Table 3. Tm values of Anti-HER2 monoclonal antibodies**

| No. | Tm1 (°C) | Tm2 (°C) |
|---|---|---|
| Trastuzumab | 68.5 | NA |
| Trastuzumab-PTM removal | 68.9 | 78.8 |
| Anti-HER2(NO3-02) | 68.0 | 77.3 |
| Anti-HER2(NO3-36) | 68.3 | 76.7 |
| Anti-HER2(NO3-46) | 71.0 | NA |

### Example 6: Affinity measurement of Anti-HER2 antibodies

ForteBio affinity measurement was performed according to the existing method (Estep, P et al., High throughput solution-based measurement of antibody-antigen affinity and epitope binning. MAbs, 2013.5(2): p.270-8). In brief, the sensor was offline equilibrated in analysis buffer for 30 minutes, then online detected for 60 seconds to establish a baseline, and the purified antibodies obtained as described above were loaded online onto the AHQ sensor. The sensor was then placed in 100nM human or cynomolgus monkey HER2 antigen (purchased from AcroBiosystems) for 5 minutes, after which the sensor was transferred to analysis buffer for dissociation for 5 minutes, and finally a 1:1 binding model was used for kinetic analysis.

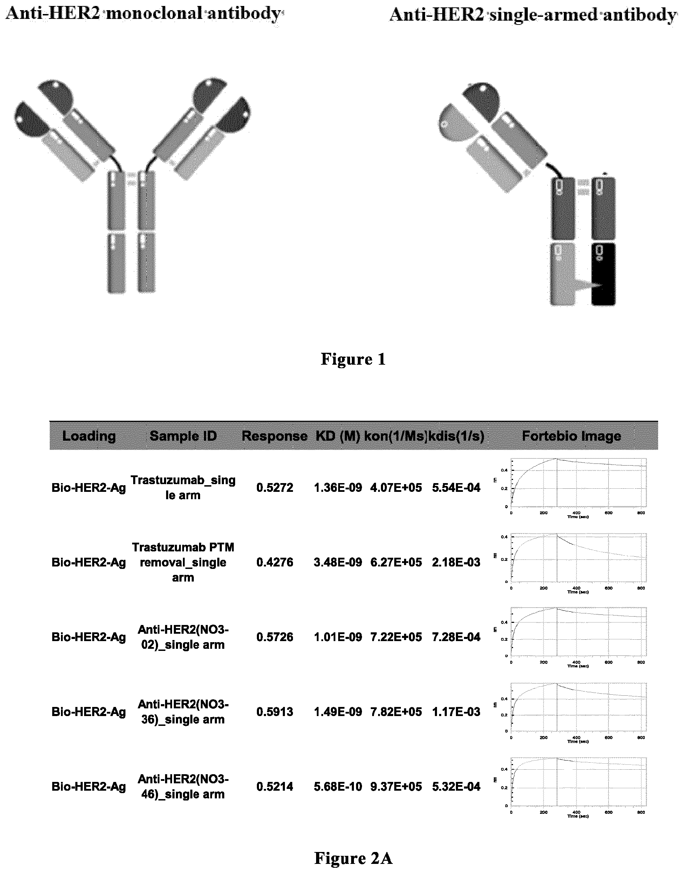

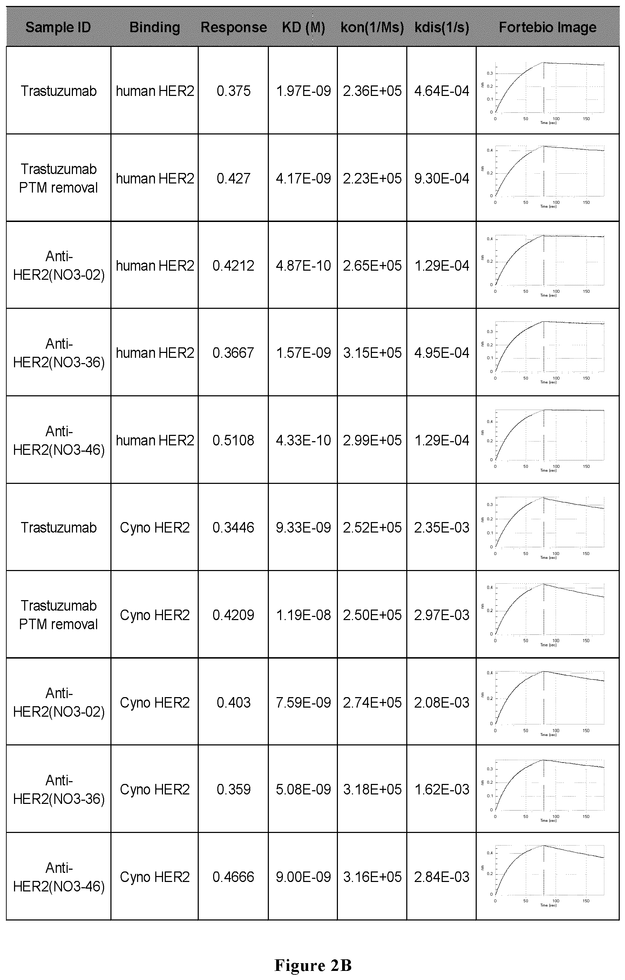

The experimental results were shown in Figures 2A and 2B. The affinity of the optimized Anti-HER2 antibody in the present invention was comparable to that of Trastuzumab before optimization. The affinity of the Trastuzumab PTM removal single-arm antibody was significantly weaker than that of the Trastuzumab single-arm antibody, while the affinity of the Anti-HER2 single-arm antibody of the present invention after PTM modification and affinity optimization for binding to human HER2 antigen was no less than that of the Trastuzumab single-arm antibody.

### Example 7: Binding of monoclonal antibody to human HER2

In this experiment, the expanded and cultured N87 cells (self-expressing HER2) were digested with 0.25% EDTA trypsin, washed once with culture medium, then adjusted to reach a cell density of 2×10⁶ cells/mL, added to a 96-well flow cytometry plate at 100µL/well, and centrifuged for later use. The gradient diluted antibodies were added to the above 96-well flow cytometry plate with cells at 100 µL/well, and incubated at 4°C for 60 min. After washing was performed twice with PBS, Goat anti-human IgG-Fc (PE) (Abcam, ab98596) diluted 1000 times with 2% BSA solution was added at 100 µL/well, and incubated at 4°C for 60 min. After washing was performed twice with PBS, the cells were resuspended by adding PBS at 100 µL/well, and detected on CytoFlex (Beckman) flow cytometer, and the corresponding mean fluorescence intensity (MFI) was calculated.

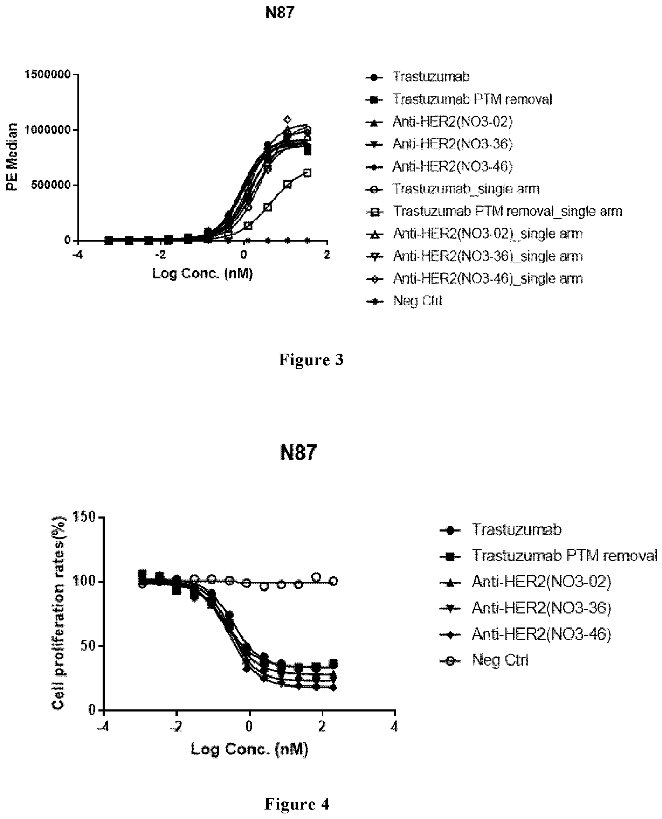

The experimental results were shown in Figure 3. The optimized Anti-HER2 antibody of the present invention had a binding activity with HER2 expressed on human gastric cancer cell N87 that was comparable to that of Trastuzumab. The cell binding activity of the Trastuzumab PTM removal single-arm antibody was significantly weaker than that of the Trastuzumab single-arm antibody, while the Anti-HER2 single-arm antibody after affinity optimization had a binding affinity with the HER2 antigen on N87 that was no less than that of the Trastuzumab single-arm antibody, and was comparable to that of the monoclonal antibody.

### Example 8. Blocking of HER2 signal-dependent cell proliferation by Anti-HER2 antibodies

In this experiment, the expanded and cultured N87 (self-expressing HER2) cells were digested with 0.25% EDTA trypsin, washed once with culture medium, and adjusted to reach a cell density of 5×10⁴ cells/mL, and added to a 96-well plate at 80µL/well for later use. The gradient diluted antibodies were added to the above 96-well plate with cells at 80 µL/well and placed in a cell culture incubator for incubation for 3 to 5 days. Finally, a kit of CellTiter-Glo^{®} Luminescent Cell Viability Assay (Promega, G7572) was used for detecting the cell viability, and then chemiluminescent signals were collected using a microplate reader.

The results were shown in Figure 4. All the optimized Anti-HER2 antibodies of the present invention could significantly inhibit the proliferation of N87 cells, and the cell proliferation inhibition effect was comparable to that of Trastuzumab monoclonal antibody.

### Example 9. Induction of ADCC effect (reporter gene) by Anti-HER2 antibodies

In this experiment, the expanded and cultured N87 (self-expressing HER2) cells at 3×10⁴/well and NFAT Luciferase/Jurkat CD16a effector cells (overexpressing CD16a and NFAT-Luc) at 1.2×10⁵/well were mixed and inoculated into a 96-well cell culture white bottom plate, and then the gradient diluted HER2 monoclonal antibodies were added to the 96-well plate and mixed, and incubated in a cell culture incubator for 6 hours. A kit of Bio-Glo luciferase assay system (Promega, G7940) was used for detecting the ADCC effect, and then chemiluminescent signals were collected using a microplate reader.

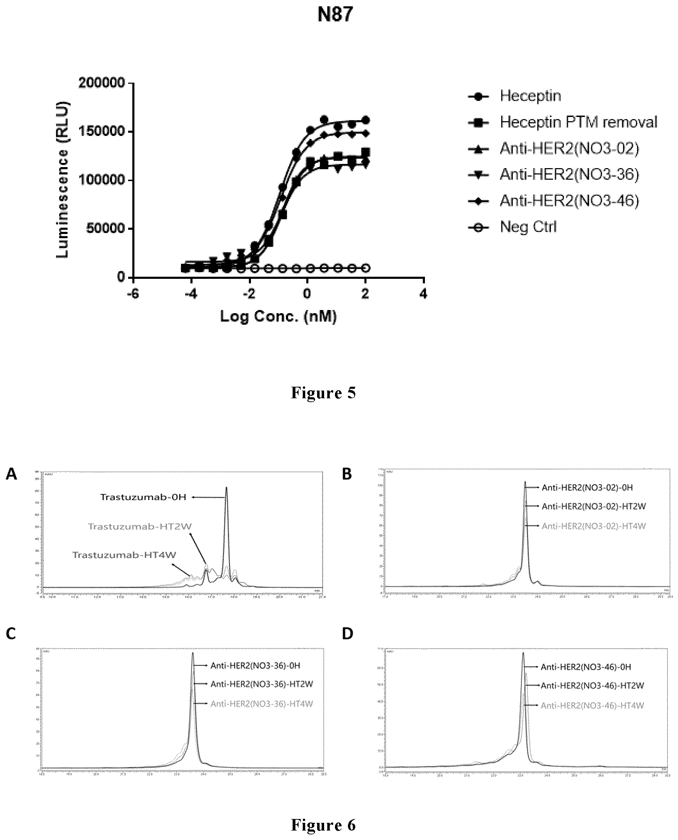

The experimental results were shown in Figure 5. The optimized Anti-HER2 monoclonal antibodies in the present invention could mediate ADCC function through HER2 expressed on N87 cells, thereby activating the CD16a-NFAT signaling pathway on Jurkat cells. In addition, the ADCC effect of Anti-HER2 (NO3-46) antibody was comparable to that of Trastuzumab antibody.

### Example 10. Stability studies of Anti-HER2 monoclonal antibodies

### 10.1. Sample processing for stability study of Anti-HER2 monoclonal antibodies

The purified protein samples were diluted to 1 mg/mL with diluent, dispensed into vials, and incubated in a 4°C refrigerator and a 40°C incubator, respectively, for 2 weeks and 4 weeks. After the samples were treated at high temperature for 2 weeks and 4 weeks, the characterization analysis and activity verification such as charge heterogeneity analysis, PTM analysis and activity verification and so on of the antibodies were performed.

### 10.2 Charge heterogeneity analysis of Anti-HER2 antibodies after high-temperature treatment

Cation exchange chromatography (CEX-HPLC) was used to determine the charge heterogeneity of Anti-HER2 antibody samples after high-temperature treatment. The CEX-HPLC method was as follows: mobile phase A: 20mM MES/MES-Na, pH6.7; mobile phase B: 20mM MES/MES-Na + 200mM NaCl, pH6.7; chromatographic conditions: detection wavelength: 280 nm; column temperature: 40°C; flow rate: 1 mL/min; detection time: 20 min; gradient: 3 to 35 min, 0% B to 100% B; chromatographic column: ProPac WCX-10 (4 × 250 mm, 10 µm).

The experimental results were shown in Figure 6. After high-temperature accelerated treatment of Trastuzumab, a significant increase in basic charge variants and acidic charge variants was observed by using CEX-HPLC. Compared with Trastuzumab, the basic charge variants and acidic charge variants of the Anti-HER2 antibodies of the present invention after PTM modification and affinity optimization did not change significantly, which indicated that their stability was significantly better.

### 10.3 PTM analysis of Anti-HER2 antibodies after high-temperature treatment

The samples of Trastuzumab and modified molecules after high-temperature accelerated treatment were taken at 200 µg and placed in 1.5 mL centrifuge tubes, respectively, added with 100 µL of protein denaturation solution (8M guanidine hydrochloride, pH6.0) and 2 µL of 1M DTT, and placed in a 37°C water bath for 30 min for protein denaturation reduction. After the water bath, instantaneous centrifugation was performed for 30 seconds, 4.4 µL of 1 mol/L IAM solution was added, vortexed for 30 seconds for mixing, underwent instantaneous centrifugation for 30 seconds, and allowed to stand in the dark for 30 minutes. After the incubation in the dark, the sample was pipetted and transferred into a 10 kDa ultrafiltration tube, 300 µL of 20 mmol/L His-HCl pH 6.0 enzyme digestion buffer was added, centrifuged at 13000 rpm/min for 15 min, desalted, and then underwent reverse centrifugation at 3000g for 3 min, the sample was recovered, and the protein concentration was determined. 40 µg of the desalted protein was taken, added with 2 µL of 0.5 mg/mL Trypsin/Lys-C mix enzyme solution (the ratio of enzyme: protein was 1: 40), supplemented with enzyme digestion buffer to 40 µL, vortexed gently for mixing, subjected to instantaneous centrifugation for a few seconds to avoid bubbles, and placed in a 37°C water bath for 4 hours. After the enzyme digestion, 2 µL of 20% formic acid aqueous solution was added to terminate the reaction, mixed well, and then centrifuged at 13000 rpm/min for 5 min, and the supernatant was taken and underwent RP-UHPLC-MS analysis.

The experimental results were shown in Table 4. Trastuzumab had deamidation and isomerization at the LC-Asn-30/HC-Asn-55/HC-Asp-102 sites, and the deamidation and isomerization of the sample were increased to a certain extent after high-temperature accelerated treatment. The results of RP-UHPLC-MS analysis showed that the degree of deamidation of Trastuzumab at the LC-Asn-30 site was significantly increased, and the deamidation or isomerization at the HC-Asn-55/HC-Asp-102 sites showed relatively less change. Compared with Trastuzumab, the Anti-HER2 antibodies of the present invention after PTM modification and affinity optimization had no detectable deamidation or isomerization at these sites, which indicated that their stability was significantly better.

**Table 4. PTM analysis results of Anti-HER2 antibodies after high-temperature treatment**

| **Sample** | **Peptide sequence** | **Site** | **Modification** | **Percentage %** |
|---|---|---|---|---|
| Trastuzumab-OH | A₂₅SQDVNTAVAWYQQKPGKAPK₄₃ | N30 (LC CDR1) | Deamidation N | 11.70% |
| Trastuzumab-HT2W | | | | 47.40% |
| Trastuzumab-HT4W | | | | 57.20% |
| Anti-HER2(NO3-02)-0H | A₂₅SQNVQGAVAWYQQKPGKAPK₄₅ | Q30 (LC CDR1) | Deamidation Q | N/A |
| Anti-HER2(NO3-02)-HT2W | | | | N/A |
| Anti-HER2(NO3-02)-HT4W | | | | N/A |
| Anti-HER2(NO3-46)-0H | A₂₅SQSVQGAVAWYQQKPG KAPK₄₅ | Q30 (LC CDR1) | Deamidation Q | N/A |
| Anti-HER2(NO3-46)-HT2W | | | | N/A |
| Anti-HER2(NO3-46)-HT4W | | | | N/A |
| Anti-HER2(NO3-36)-0H | A₂₅SNYVQTAVAWYQQKPG KAPK₄₅ | Q30 (LC CDR1) | Deamidation Q | N/A |
| Anti-HER2(N03-36)-HT2W | | | | N/A |
| Anti-HER2(NO3-36)-HT4W | | | | N/A |
| Trastuzumab-OH | I₅₁YPTNGYTR₅₉ | N55 (HC CDR2) | Deamidation N | 2.10% |
| Trastuzumab-HT2W | | | | 4.10% |
| Trastuzumab-HT4W | | | | 5.30% |
| Anti-HER2(NO3-02)-0H | I₅₁YPTQGYTR₅₉ | Q55 (LC CDR2) | Deamidation Q | N/A |
| Anti-HER2(NO3-02)-HT2W | | | | N/A |
| Anti-HER2(NO3-02)-HT4W | | | | N/A |
| Anti-HER2(NO3-46)-0H | I₅₁YPTQGYTR₅₉ | Q55 (LC CDR2) | Deamidation Q | N/A |
| Anti-HER2(NO3-46)-HT2W | | | | N/A |
| Anti-HER2(NO3-46)-HT4W | | | | N/A |
| Anti-HER2(NO3-36)-0H | I₅₁YPTQGYTR₅₉ | Q55 (LC CDR2) | Deamidation Q | N/A |
| Anti-HER2(NO3-36)-HT2W | | | | N/A |
| Anti-HER2(NO3-36)-HT4W | | | | N/A |
| Trastuzumab-OH | W₉₉GGDGFYAMDYWGQGTLVTVSS ASTKGPSVFPLAPSSK₁₃₆ | D102 (HC CDR3) | Succinimide D | 3.50% |
| Trastuzumab-HT2W | | | | 3.70% |
| Trastuzumab-HT4W | | | | 3.50% |
| Anti-HER2(NO3-02)-0H | W₉₉GGEGFYAMDYWGQGTLVTVSS ASTKGPSVFPLAPSSK₁₃₆ | E102 (HC CDR3) | Succinimide E | N/A |
| Anti-HER2(NO3-02)-HT2W | | | | N/A |
| Anti-HER2(NO3-02)-HT4W | | | | N/A |
| Anti-HER2(NO3-46)-0H | W₉₉GGEGFYAMDYWGQGTLVTVSS ASTKGPSVFPLAPSSK₁₃₆ | E102 (HC CDR3) | Succinimide E | N/A |
| Anti-HER2(NO3-46)-HT2W | | | | N/A |
| Anti-HER2(NO3-46)-HT4W | | | | N/A |
| Anti-HER2(NO3-36)-0H | W₉₉GGEGFYAMDYWGQGTLVTVSS ASTKGPSVFPLAPSSK₁₃₆ | E102 (HC CDR3) | Succinimide E | N/A |
| Anti-HER2(NO3-36)-HT2W | | | | N/A |
| Anti-HER2(NO3-36)-HT4W | | | | N/A |

| | | | | |
|---|---|---|---|---|
| N/A means not detected | | | | |

### 10.4 Activity verification of Anti-HER2 antibodies after high-temperature treatment

ForteBio affinity measurement was performed according to the existing method (Estep, P et al., High throughput solution-based measurement of antibody-antigen affinity and epitope binning. MAbs, 2013.5(2): p.270-8). In brief, the sensor was offline equilibrated in analysis buffer for 30 minutes, then online detected for 60 seconds to establish a baseline, and the purified antibodies obtained as described above were loaded online onto the AHQ sensor. The sensor was then placed in 100nM human HER2 antigen (purchased from AcroBiosystems) for 5 minutes, after which the sensor was transferred to analysis buffer for dissociation for 5 minutes, and finally a 1:1 binding model was used for kinetic analysis.

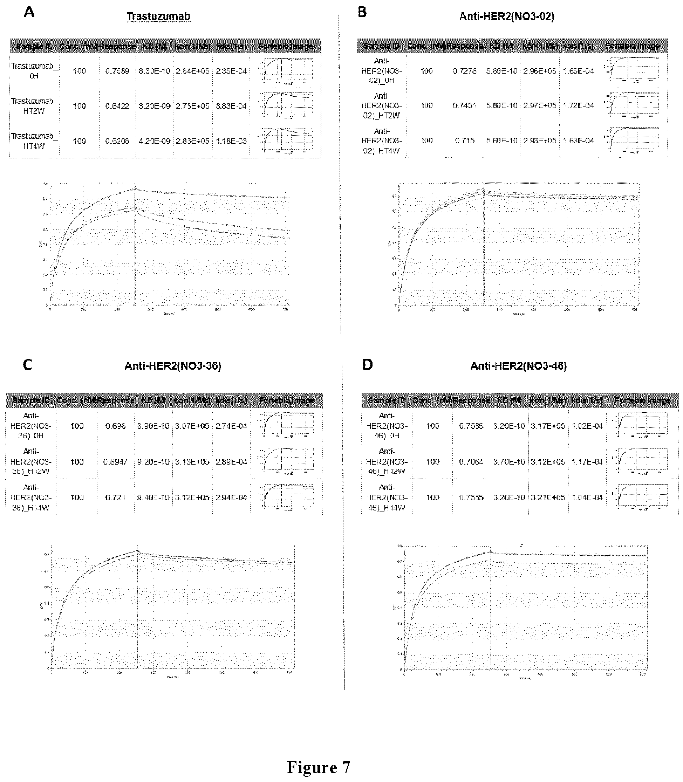

The experimental results were shown in Figure 7. After 2 weeks and 4 weeks of high-temperature treatment, the affinity of Trastuzumab for binding to human HER2 antigen showed a significant decrease, and the longer the high-temperature treatment time, the weaker its binding activity. Compared with Trastuzumab, the Anti-HER2 antibodies of the present invention after PTM modification and affinity optimization had no significant change in their binding activity to HER2 antigen before and after high-temperature treatment, which indicated that their stability was significantly better.

Although the specific embodiments of the present invention have been described in detail, those skilled in the art will understand that various modifications and changes can be made to the details based on all the teachings that have been disclosed, and these changes are within the scope of protection of the present invention. The entirety of the present invention is given by the appended claims and any equivalents thereof.

## Claims

1. An antibody or antigen-binding fragment thereof capable of specifically binding to HER2, the antibody or antigen-binding fragment thereof comprising:
(1) the following 3 heavy chain variable region (VH) complementarity determining regions (CDRs):
(a) VH CDR1 having a structure represented by GFNIKDTY (SEQ ID NO: 10);
(b) VH CDR2 having a structure represented by IYPTQGYT (SEQ ID NO: 11);
(c) VH CDR3 having a structure represented by SRWGGEGFYAMDY (SEQ ID NO: 12);
and/or,
(2) the following 3 light chain variable region (VL) complementarity determining regions (CDRs):
(d) VL CDR1 having a structure represented by X₁X₂VQX₃A (SEQ ID NO: 33);
(e) VL CDR2 having a structure represented by SAS (SEQ ID NO: 19, SEQ ID NO: 23 or SEQ ID NO: 27);
(f) VL CDR3 having a structure represented by QQHX₄X₅TPPT (SEQ ID NO: 34);
wherein:
X₁ is selected from amino acid residues Q and N;
X₂ is selected from amino acid residues N, Y and S;
X₃ is selected from amino acid residues G and T;
X₄ is selected from amino acid residues Y, F and S;
X₅ is selected from amino acid residues S, M and T.

2. The antibody or antigen-binding fragment thereof according to claim 1, comprising:
a VH CDR1 as set forth in SEQ ID NO: 10, a VH CDR2 as set forth in SEQ ID NO: 11, and a VH CDR3 as set forth in SEQ ID NO: 12; and,
a VL CDR1 as set forth in any one of SEQ ID NOs: 18, 22 and 26, a VL CDR2 as set forth in any one of SEQ ID NOs: 19, 23 and 27, and a VL CDR3 as set forth in any one of SEQ ID NOs: 20, 24 and 28.

3. The antibody or antigen-binding fragment thereof according to any one of claims 1 to 2, comprising:
(1) a VH CDR1 as set forth in SEQ ID NO: 10, a VH CDR2 as set forth in SEQ ID NO: 11, and a VH CDR3 as set forth in SEQ ID NO: 12; and a VL CDR1 as set forth in SEQ ID NO: 18, a VL CDR2 as set forth in SEQ ID NO: 19, and a VL CDR3 as set forth in SEQ ID NO: 20; or,
(2) a VH CDR1 as set forth in SEQ ID NO: 10, a VH CDR2 as set forth in SEQ ID NO: 11, and a VH CDR3 as set forth in SEQ ID NO: 12; and a VL CDR1 as set forth in SEQ ID NO: 22, a VL CDR2 as set forth in SEQ ID NO: 23, and a VL CDR3 as set forth in SEQ ID NO: 24; or,
(3) a VH CDR1 as set forth in SEQ ID NO: 10, a VH CDR2 as set forth in SEQ ID NO: 11, and a VH CDR3 as set forth in SEQ ID NO: 12; and a VL CDR1 as set forth in SEQ ID NO: 26, a VL CDR2 as set forth in SEQ ID NO: 27, and a VL CDR3 as set forth in SEQ ID NO: 28.

4. The antibody or antigen-binding fragment thereof according to any one of claims 1 to 3, comprising:
a VH having a sequence as set forth in SEQ ID NO: 9 or a variant thereof, and a VL having a sequence as set forth in any one of SEQ ID NOs: 17, 21 and 25 or a variant thereof;
wherein, the variant has a sequence having a substitution, deletion or addition of one or more amino acids (e.g., a substitution, deletion or addition of 1, 2, 3, 4 or 5 amino acids), or a sequence identity of at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived; preferably, the substitution is a conservative substitution.

5. The antibody or antigen-binding fragment thereof according to any one of claims 1 to 4, comprising:
(1) a VH having a sequence as set forth in SEQ ID NO: 9, and a VL having a sequence as set forth in SEQ ID NO: 17; or,
(2) a VH having a sequence as set forth in SEQ ID NO: 9, and a VL having a sequence as set forth in SEQ ID NO: 21; or,
(3) a VH having a sequence as set forth in SEQ ID NO: 9, and a VL having a sequence as set forth in SEQ ID NO: 25.

6. The antibody or antigen-binding fragment thereof according to any one of claims 1 to 5, further comprising a constant region derived from a human immunoglobulin;
preferably, the heavy chain of the antibody or antigen-binding fragment thereof comprises a heavy chain constant region derived from a human immunoglobulin (e.g., IgG1, IgG2, IgG3, or IgG4);
preferably, the light chain of the antibody or antigen-binding fragment thereof comprises a light chain constant region derived from a human immunoglobulin (e.g., κ or λ).

7. The antibody or antigen-binding fragment thereof according to any one of claims 1 to 6, further comprising a heavy chain constant region as set forth in SEQ ID NO: 29 and/or a light chain constant region as set forth in SEQ ID NO: 31.

8. The antibody or antigen-binding fragment thereof according to any one of claims 1 to 7, wherein the antigen-binding fragment is selected from the group consisting of Fab, Fab', (Fab')₂, Fv, disulfide-bonded Fv, scFv, diabody and single domain antibody (sdAb).

9. A single-arm antibody, comprising:
(1) a first peptide chain, wherein the first peptide chain comprises 3 heavy chain variable region (VH) complementarity determining regions (CDRs), and the 3 heavy chain variable region (VH) complementarity determining regions (CDRs) are defined as those in any one of claims 1 to 3,
(2) a second peptide chain, wherein the second peptide chain comprises 3 light chain variable region (VL) complementarity determining regions (CDRs), and the 3 light chain variable region (VL) complementarity determining regions (CDRs) are defined as those in any one of claims 1 to 3, and,
(3) a third peptide chain, wherein the third peptide chain is capable of forming a dimer with the first peptide chain;
preferably, the first peptide chain comprises a heavy chain variable region (VH), and the heavy chain variable region (VH) is defined as that in any one of claims 4 to 5;
preferably, the second peptide chain comprises a light chain variable region (VL), and the light chain variable region (VL) is defined as that in any one of claims 4 to 5.

10. The single-arm antibody according to claim 9, wherein the second peptide chain further comprises a constant region derived from a human immunoglobulin;
preferably, the second peptide chain comprises a light chain constant region derived from a human immunoglobulin (e.g., κ or λ);
preferably, the second peptide chain comprises a light chain constant region as set forth in SEQ ID NO: 31.

11. The single-arm antibody according to any one of claims 9 to 10, wherein the single-arm antibody further has at least one technical feature selected from the following items (1) to (2):
(1) the first peptide chain further comprises a constant region derived from a human immunoglobulin; preferably, the constant region derived from a human immunoglobulin is a heavy chain constant region derived from a human immunoglobulin (e.g., IgG1, IgG2, IgG3 or IgG4); preferably, the heavy chain constant region has a first modification to promote dimerization of the first peptide chain and the third peptide chain;
(2) the third peptide chain comprises an Fc domain monomer; preferably, the Fc domain monomer is an Fc domain monomer of IgG, such as an Fc domain monomer of IgG1, IgG2, IgG3 or IgG4; preferably, the Fc domain monomer has a second modification to promote dimerization of the third peptide chain and the first peptide chain;
preferably, any one of the first modification and the second modification is a "knob" modification, and the other is a "hole" modification to form a "knob-into-hole" modification to promote dimerization of the first peptide chain and the third peptide chain;
preferably, the first modification is a "knob" modification, and the second modification is a "hole" modification to form a "knob-into-hole" modification to promote dimerization of the first peptide chain and the third peptide chain;
preferably, the heavy chain constant region comprises an amino acid sequence as set forth in SEQ ID NO: 30, and the Fc domain monomer comprises an amino acid sequence as set forth in SEQ ID NO: 32.

12. An isolated nucleic acid molecule, encoding the antibody or antigen-binding fragment thereof according to any one of claims 1 to 8, or its heavy chain variable region and/or light chain variable region; or, encoding the single-arm antibody according to any one of claims 9 to 11, or its heavy chain variable region and/or light chain variable region.

13. A vector, comprising the nucleic acid molecule according to claim 12; preferably, the vector is a cloning vector or an expression vector.

14. A host cell, comprising the nucleic acid molecule according to claim 12 or the vector according to claim 13.

15. A method for preparing the antibody or antigen-binding fragment thereof according to any one of claims 1 to 8 or the single-arm antibody according to any one of claims 9 to 11, comprising culturing the host cell according to claim 14 under conditions that allow the expression of the antibody or antigen-binding fragment thereof or the single-arm antibody, and recovering the antibody or antigen-binding fragment thereof or the single-arm antibody from a culture of the cultured host cell.

16. A pharmaceutical composition, comprising the antibody or antigen-binding fragment thereof according to any one of claims 1 to 8 or the single-arm antibody according to any one of claims 9 to 11, and optionally comprising pharmaceutically acceptable carriers and/or excipients.

17. Use of the antibody or antigen-binding fragment thereof according to any one of claims 1 to 8, the single-arm antibody according to any one of claims 9 to 11, the isolated nucleic acid molecule according to claim 12, the vector according to claim 13, or the host cell according to claim 14, in the manufacture of a medicament for activating HER2, increasing immune cell activity, enhancing immune response, and/or preventing and/or treating a tumor or infection in a subject;
preferably, the immune cell is a T cell, a B cell, a DC cell, a macrophage, and/or an NK cell;
preferably, the immune response is a HER2-mediated immune response;
preferably, the subject is a mammal, such as a human;
preferably, the antibody or antigen-binding fragment thereof or the single-arm antibody is used alone or in combination with an additional pharmaceutically active agent.

18. A method for enhancing immune response, and/or preventing and/or treating a tumor or infection in a subject, wherein the method comprises: administering to the subject in need thereof an effective amount of the antibody or antigen-binding fragment thereof according to any one of claims 1 to 8, or the single-arm antibody according to any one of claims 9 to 11, or the pharmaceutical composition according to claim 16;
preferably, the immune response is a HER2-mediated immune response;
preferably, the subject is a mammal, such as a human.

19. A conjugate, comprising the antibody or antigen-binding fragment thereof according to any one of claims 1 to 8 or the single-arm antibody according to any one of claims 9 to 11, and optionally a detectable label conjugated to the antibody or antigen-binding fragment thereof or the single-arm antibody;
preferably, the detectable label is selected from the group consisting of enzyme (e.g., horseradish peroxidase or alkaline phosphatase), chemiluminescent reagent (e.g., acridinium ester compound, luminol and derivative thereof, or ruthenium derivative), fluorescent dye (e.g., fluorescein or fluorescent protein), radionuclide and biotin.

20. A kit, comprising the antibody or antigen-binding fragment thereof according to any one of claims 1 to 8, or the single-arm antibody according to any one of claims 9 to 11, or the conjugate according to claim 19;
preferably, the kit comprises the conjugate according to claim 19;
preferably, the kit comprises the antibody or antigen-binding fragment thereof according to any one of claims 1 to 8, and a second antibody capable of specifically recognizing the antibody or antigen-binding fragment thereof; optionally, the second antibody further comprises a detectable label, such as an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide or biotin;
preferably, the kit comprises the single-arm antibody according to any one of claims 9 to 11, and a second antibody capable of specifically recognizing the single-arm antibody; optionally, the second antibody further comprises a detectable label, such as an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide or biotin.

21. A method for detecting the presence or level of HER2 in a sample, comprising using the antibody or antigen-binding fragment thereof according to any one of claims 1 to 8, or the single-arm antibody according to any one of claims 9 to 11, or the conjugate according to claim 19;
preferably, the method is an immunological assay, such as immunoblotting, enzyme immunoassay (e.g., ELISA), chemiluminescent immunoassay, fluorescent immunoassay or radioimmunoassay;
preferably, the method comprises using the conjugate according to claim 19;
preferably, the method comprises using the antibody or antigen-binding fragment thereof according to any one of claims 1 to 8, and the method further comprises using a second antibody carrying a detectable label (e.g., an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide or biotin) to detect the antibody or antigen-binding fragment thereof;
preferably, the method comprises using the single-arm antibody according to any one of claims 9 to 11, and the method further comprises using a second antibody carrying a detectable label (e.g., an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide or biotin) to detect the single-arm antibody.

22. Use of the antibody or antigen-binding fragment thereof according to any one of claims 1 to 8, or the single-arm antibody according to any one of claims 9 to 11, or the conjugate according to claim 19, in the manufacture of a detection reagent for detecting the presence or level of HER2 in a sample;
preferably, the detection reagent detects the presence or level of HER2 in a sample by the method according to claim 21;
preferably, the sample is a cell sample (e.g., an immune cell) from a subject (e.g., a mammal, preferably a human or a cynomolgus monkey).
